# EUROPEAN PATENT SPECIFICATION

(11) **EP 0 851 862 B1**
(45) Date of publication and mention of the grant of the patent: **10.09.2003**
(21) Application number: 96927581.7
(22) Date of filing: 23.07.1996
(51) Int. Cl.: C07D 277/84, C07D 417/12, A61K 31/425

(54) **1(HETERO)ARYL-4-(CONDENSED THIAZOL-2-YLALKYL)-PIPERAZINE DERIVATIVES, THEIR PREPARATION AND THEIR USE IN THE TREATMENT OF 5-HT1A-RECEPTOR MEDIATED DISORDERS**
1-(HETERO)ARYL-4-(KONDENSIERTES THIAZOL-2-YLALKYL)-PIPERAZIN DERIVATE, DEREN HERSTELLUNG UND DEREN VERWENDUNG IN DER BEHANDLUNG VON 5-HT1A-REZEPTOR VERMITTELTEN KRANKHEITEN
DERIVES 1-(HETERO)ARYL-4-(THIAZOL-2-YLALKYL CONDENSE)-PIPERAZINES, LEUR PREPARATION ET LEUR UTILISATION DANS LE TRAITEMENT DE TROUBLES INDUITS PAR LE RECEPTEUR DE 5-HT1A

(30) Priority: 24.08.1995 GB 9517381
(43) Date of publication of application: 08.07.1998
(73) Proprietor: Pharmacia Italia S.p.A., 20152 Milano (IT)
(72) Inventor: MC ARTHUR, Robert, I-20143 Milan (IT); PERRONE, Roberto, I-70126 Bari (IT); FORNARETTO, Maria, Gioia, I-20153 Milan (IT)
(86) International application number: EP9603240
(87) International publication number: WO97008159

(56) References cited:
- EP-A- 0 279 598
- EP-A- 0 361 271
- EP-A- 0 434 561
- WO-A-94/06789
- DE-A- 2 717 415
- GB-A- 1 164 572
- US-A- 2 912 434
- US-A- 2 942 003
- JOURNAL OF MEDICINAL CHEMISTRY, vol. 32, no. 8, August 1989, pages 1921-1926, XP002018027 GLENNON R.A. ET AL.: "N-(Phthalimidoalkyl) derivatives of serotonergic agents: A common interaction at 5-HT1A serotonin binding sites?"
- JOURNAL OF MEDICINAL CHEMISTRY, vol. 34, no. 6, June 1991, pages 1860-1866, XP002018028 LOWE J.A. III ET AL.: "1-Naphthylpiperazine derivatives as potential atypical antipsychotic agents"
- IL FARMACO, vol. 50, no. 2, February 1995, pages 77-82, XP000605205 PERRONE R. ET AL.: "Conformationally restricted thiazole derivatives as novel class of 5-HT3 receptor ligands" cited in the application
- MEDICINAL CHEMISTRY RESEARCH, vol. 4, no. 1, 1 January 1994, pages 16-84, XP000604196 SAUDOU F. & HEN R.: "5-HT receptor subtypes: Molecular and functional diversity" cited in the application
- CHEMICAL ABSTRACTS, vol. 73, no. 9, 31 August 1970 Columbus, Ohio, US; abstract no. 45502p, YAMANE K.: "Benzocycloheptathiazoles" page 325; column 1; XP002018029 & JP,A,45 014 068 (JAPANESE RESEARCH INSTITUTE FOR PHOTOSENSITIZING DYES CO., LTD.) 20 May 1970

## Description

The present invention relates to aryl and heteroaryl piperazine derivatives, to a process for their preparation and to pharmaceutical compositions comprising them. These compounds possess central serotoninergic activity and can be useful in the treatment of central nervous system disorders.

Serotonin (5-hydroxytryptamine, 5-HT) is a biogenic amine which was first discovered in the gut. 5-HT was later shown to correspond to a vasotonic substance found in the serum and was therefore called serotonin. Serotonin is also found in the brain and is involved in a wide range of behaviours such as sleep, appetite, pain perception, locomotion, thermoregulation and sexual activity.
Furthermore, serotonergic drugs are used in the treatment of a number of pathological states such as migraine, depression and anxiety.
The multiple actions of serotonin are mediated by the specific interaction of this amine with several receptors. Pharmacological and physiological studies identified distinct receptors which were designated 5-HT_{1A}, 5-HT_{1B}, 5-HT_{1C} , 5-HT_{1D}, 5-HT₂, 5-HT₃ and 5-HT₄ (Med. Chem. Res., 1994, 4, 16-84).

It is known that the 5-HT_{1A} receptor sub-family seems to be important for many functions in humans. For instance, altered functions of this receptor sub-family is involved in the genesis of a number of pathological conditions such as, e.g., anxiety, depression, psychosis, abnormality of sleep and feeding, organic mental diseases and alteration of the blood pressure.
Therefore, pharmaceutical compounds able to act on 5-HT_{1A} receptor sites may be useful in the treatment of disorders which depend on serotonin action at the level of 5HT_{1A} receptors.

Perrone et al. disclosed in "Il Farmaco 50(2) 77-82, 1995", N-methyl-N-tricyclic naphtho-[1,2-d]thiazol piperazine derivatives, which were found to possess 5-HT₃ receptor binding affinity whereas they displayed no affinity versus D₂, 5-HT₁ and 5-HT₂ receptors.
In view of the above results, a person skilled in the art, seeking to prepare compounds which possess 5-HT_{1A} receptor affinity, would be taught away from considering tricyclic naphtho-[1,2-d]thiazol-piperazine structures.

It has now surprisingly been found that N-aryl and heteroaryl-piperazin-naphtho[1,2-d]thiazol derivatives, which show a remarkable structural similarity with the above compounds disclosed by Perrone, exhibit a noticeable degree of affinity towards 5-HT_{1A} receptor sites.

Accordingly, the present invention provides an aryl or heteroaryl piperazine derivative of formula (I) 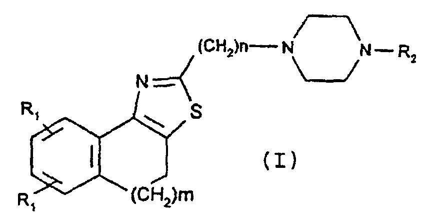 wherein
R₁ is hydrogen or a C₁-C₆ alkoxy group;
m is zero 1 or 2;
n is an integer from 1 to 4;
R₂ is an unsubstituted phenyl ring, a phenyl ring substituted by a C₁-C₃ alkoxy group, an unsubstituted pyridyl ring, an unsubstituted pyrimidinyl ring or an unsubstituted pyrazinyl ring;
and the pharmaceutically acceptable salts thereof.

Examples of specific compounds of the invention are the following:
(1) 6-Methoxy-2-{4-(4-(2-methoxy-phenyl)-piperazin-1-yl]-butyl}-4,5-dihydro-naphtho[1,2-d]thiazole hydrochloride;
(2) 2-[(4-phenyl-piperazin-1-yl)-methyl]-4,5 dihydronaphtho[1,2-d]thiazole hydrocloride;
(3) 2-[2-(4-phenyl-piperazin-1-yl-ethyl]-4,5-dihydronaphtho[1,2-d]thiazole hydrocloride;
(4) 2-[3-(4-phenyl-piperazin-1-yl)-propyl]-4,5-dihydronaphtho[1,2-d]thiazole hydrocloride;
(5) 2-[4-(4-phenyl-piperazin-1-yl)-butyl]-4,5-dihydronaphtho[1,2-d]thiazole hydrocloride;
(6) 7-Methoxy-2-{3-[4- (2-methoxy-phenyl) -piperazin-1-yl]-propyl}-4,5-dihydro-naphtho[1,2-d]thiazole hydrochloride;
(7) 7-Methoxy-2-[4-(4-phenyl-piperazin-1-yl)-butyl]-4,5-dihydro-naphtho[1,2-d]thiazole hydrochloride;
(8) 7-Methoxy-2-{4-[4-(2-methoxy-phenyl)-piperazin-1-yl]-butyl}-4,5-dihydro-naphtho[1,2-d]thiazole hydrochloride;
(9) 7-Methoxy-2-{4-[4-(2-pyridyl)-piperazin-1-yl]-butyl}-4,5-dihydro-naphtho[1,2-d]thiazole hydrochloride;
(10) 6-Methoxy-2-[4-(4-phenyl-piperazin-1-yl)-butyl]-4,5-dihydro-naphtho[1,2-d]thiazole hydrochloride;
(11) 5-Methoxy-2-[4-(4-phenyl-piperazin-1-yl)-butyl]-4,5-dihydro-naphtho[1,2-d]thiazole hydrochloride;
(12) 5-Methoxy-2-{4-[4-(2-pyridyl)-piperazin-1-yl]-butyl}-4,5-dihydro-naphtho[1,2-d]thiazole hydrochloride; and
(13) 5-Methoxy-2-{4-[4-(2-methoxy-phenyl)-piperazin-1-yl]-butyl}-4,5-dihydro-naphtho[1,2-d]thiazole hydrochloride.

The compounds of the invention can be obtained by a process comprising:
a) condensing a compound of formula (II) with a compound of formula (III) 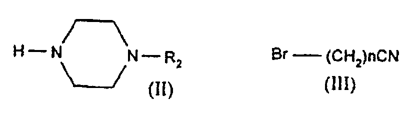 wherein n and R₂ are as defined in claim 1 so obtaining a compound of formula (IV) 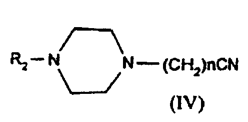 wherein n and R₂ are as defined above;
b) hydrolysing the compound of formula (IV) under acid conditions to obtain a compound of formula (V) 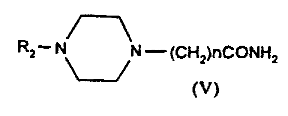 wherein n and R₂ are as defined above;
c) reacting the compound of formula (V) with P₂S₅ or the Lawesson reagent [2,4-bis(4-methoxyphenyl)-1,3-dithia-2,4-diphosphetane-2,4-disulfide], to obtain a compound of formula (VI) 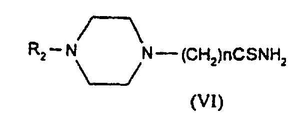 wherein R₂ and n are as defined above;
d) condensing the compound of formula (VI) with a compound of formula (VII) 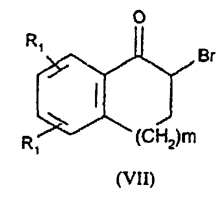 wherein R₁ is hydrogen, C₁-C₆ alkoxy, and m is zero or an integer from 1 to 2, and, if desired, converting the resulting compounds of formula (I) into another compound of formula (I) ; and/or if desired converting a compound of formula (I) into a pharmaceutically acceptable salt thereof.

The condensation described as step a) may be carried out, for example,by reaction of a compound of formula (II) with a compound of formula (III) in a solvent such as, e.g., dimethylformamide (DMF) or tetrahydrofurane about 30° to about 75°C.

The hydrolysis described as step b), which is performed as a controlled acid hydrolysis, may be carried out, for example,by pouring a compound of formula (IV) into ice cooled concentrated sulfuric acid for a time varying from about 25 to about 50 minutes.

The reaction described as step c) may be carried out, for example, by heating a compound of formula (V) with the Lawesson reagent in toluene at reflux for a time varying from about 3 to about 7 hours, or treating a compound of formula (V) with P₂S₅ in pyridine at reflux for a time varying from about 4 to about 8 hours.

The condensation described as step d) may be carried out, for example, by reacting a compound of formula (VI) with a compound of formula (VII) in a suitable solvent such as, e.g., THF, ethanol, diethyl ether or dioxane, at a temperature ranging from about 35°C to about 110°C.

The optional conversion of a compound of formula (I) into a salt thereof may be carried out, for example, by adding a stoichiometric amount of a suitable pharmaceutically acceptable acid dissolved in a siutable solvent such as, e.g., diethyl ether, ethanol or methanol, to a solution of the base.The salt can be recovered by concentration of the solution.

The compounds of the present invention possess high affinity towards 5-HT_{1A} receptor sites having agonist or antagonist activity at central level.
They are therefore useful in the treatment of disorders which depend on serotonin action at the level of 5HT_{1A} receptor sites. For example, they can be useful in the treatment of anxiety, depression, schizophrenia, pain (Drug of the future, 1988, 13(5), 429-437), age associated memory impairment, Alzheimer's disease, feeding and sexual disorders.
By virtue of their neuroprotective activity, the compounds of the present invention may also be useful to alleviate neuronal damage following cerebral ischemia (Stroke, 1990,21(4), 161-167).

A human or animal body may thus be treated by a method which comprises the administration thereto of a pharmaceutically effective amount of a compound of formula (I) or salt thereof. The condition of the human or animal can thereby be improved.

The affinity of the compounds of the present invention towards 5-HT_{1A} receptor sites can be assessed by evaluating the binding profile of the compounds of formula (I).
As an example, a representative number of compounds of formula (I), namely compounds (1) to (13) listed above, were tested according to the method reported in Journal of Neurochemistry, 44, 1685, (1985), and Molecular Pharmacology, 21, 301, (1981).
The obtained results are reported in Table 1 below.

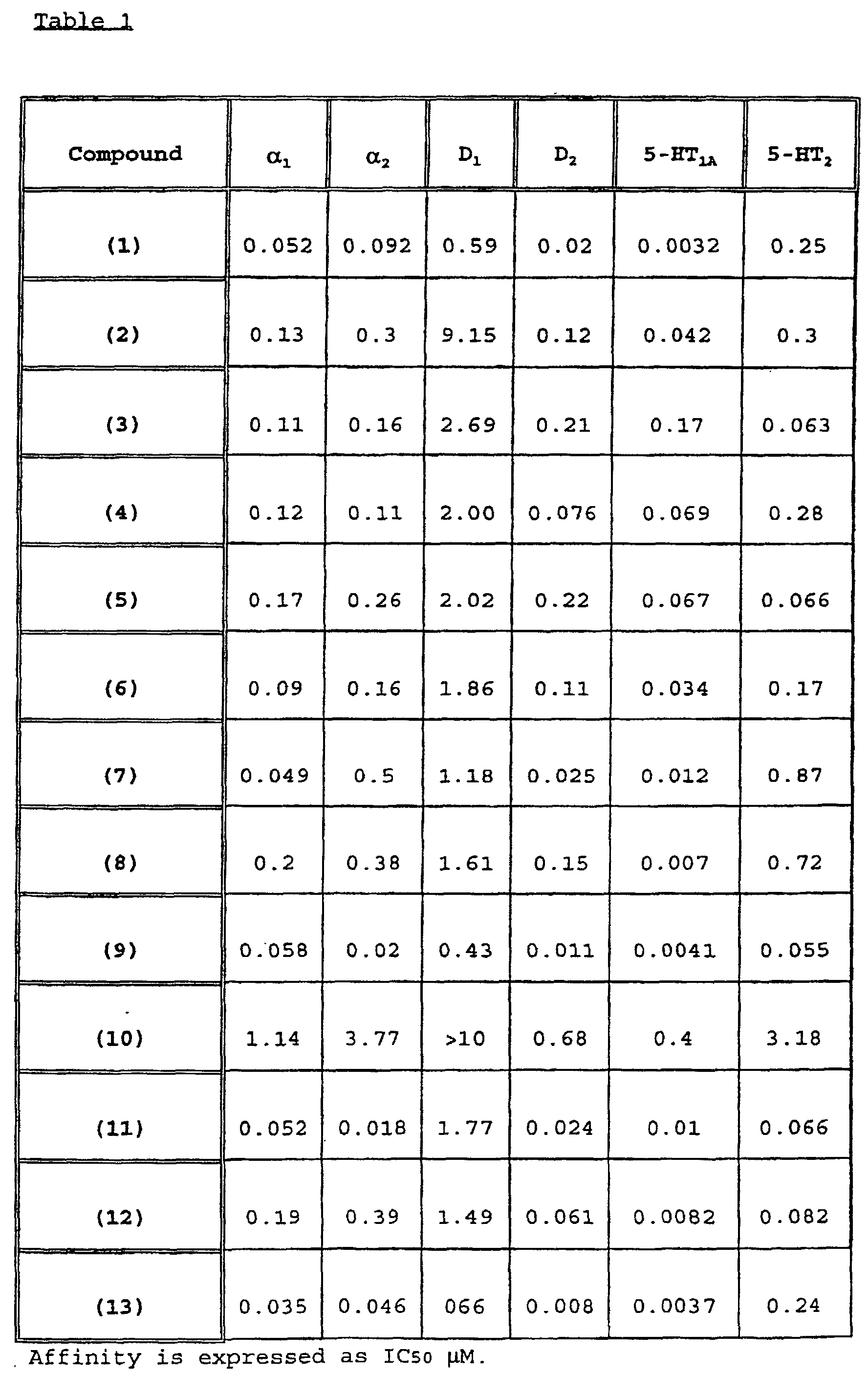

The tabulated data clearly demonstrate the high affinity of the compounds of formula (I) towards 5HT_{1A} receptor sites.
A mammal, e.g. a human or animal, may thus be treated by a method which comprises administering thereto a pharmaceutically effective amount of a compound of formula (I) as defined above.

The compounds of the invention can be administered in a variety of dosage forms, e.g. orally, in the form of tablets, capsules, sugar or film coated tablets, liquid solutions or suspensions; rectally, in the form of suppositories;
parenterally, e.g. intramuscularly, or by intravenous injection or infusion; topically, e.g. in the form of creams. Preferably they are administered by oral or parenteral route, more preferably by oral route.
The dosage depends on the age, weight, conditions of the patient and administration route. For example, a suitable dosage for oral administration to adult humans may be from 1mg to 1000mg per kg per day, preferably from 50mg to 500mg per kg.

The present invention also provides pharmaceutical compositions comprising, as an active ingredient, a compound of formula (I) as defined above or a pharmaceutically acceptable salt thereof, in mixture with a pharmaceutically acceptable excipient (which can be diluent and/or carrier).
The pharmaceutical compositions containing the compounds of the invention are usually prepared following conventional methods and are administered in a pharmaceutically suitable form.
For example, the solid oral forms may contain, together with the active compound, diluents, e.g., lactose, dextrose, saccharose, cellulose, corn starch or potato starch; lubricants, e.g. silica, talc, stearic acid, magnesium or calcium stearate, and/or polyethylene glycols; binding agents, e.g.starches, arabic gums, gelatin, methylcellulose, carboxymethylcellulose or polyvinyl pyrrolidone;disaggregating agents, e.g. a starch, alginic acid, alginates or sodium starch glycolate; effervescing mixtures;dyestuffs; sweeteners; wetting agents such as lecithin, polysorbates, laurylsulphates; and, in general, non-toxic and pharmacologically inactive substances used in pharmaceutical formulations. Said pharmaceutical preparations may be manufactured in known manner, for example, by means of mixing granulating, tabletting, sugar-coating, or film-coating processes.
The liquid dispersions for oral administration may be, e.g. syrups, emulsions and suspension.
The syrups may contain as carrier, for example, saccharose or saccharose with glycerine and/or mannitol and/or sorbitol; in particular a syrup to be administered to diabetic patients can contain as carriers only products not metabolizable to glucose, or metabolizable in very small amount to glucose, for example sorbitol.
The suspensions and the emulsions may contain as carrier, for example,a natural gum,agar sodium alginate, pectin pectin,methylcellulose,caroxymethylcellulose, or polyvinyl alcohol.
The suspensions or solutions for intramuscular injections may contain, together with the active compound, a pharmaceutically acceptable carrier, e.g. sterile water, olive oil, ethyl oleate, glycol, e.g. propylene glycol and if desired, a suitable amount of lidocaine hydrochloride.
The solutions for intravenous injections or infusions may contain as carrier, for example, sterile water or preferably they may be in the form of sterile, aqueous, isotonic saline solutions.
The suppositories may contain together with the active compound a pharmaceutically acceptable carrier, e.g. cocoa-butter, polyethylene glycol, a polyoxyethylene sorbitan fatty acid ester surfactant or lecithin.
Conventional carriers may be used for topical formulations.

The following examples, representative of the compounds of the invention, illustrate the invention.
The number into bracket reported before the chemical name of the compounds prepared according to the following examples, corresponds to the preferred compounds listed on pages 5 and 6 of the present specification.

### Example 1

### (1) 6-Methoxy-2-{4-(4-(2-methoxy-phenyl)-piperazin-1-yl]-butyl}-4,5-dihydro-naphtho[1,2-d]thiazole hydrochloride

A stirred suspension of 1.92 g of 1-(2-methoxyphenyl)piperazine and 2.16 g of 5-bromopentane nitrile and 1.4 g of potassium carbonate in 50 ml of acetonitrile was refluxed for 1 hour.
After cooling, the solvent was removed and the residue taken up in ethyl acetate was washed with brine.
After drying, the crude reaction mixture was chromatographed on silica gel eluting with ethyl acetate/dichloromethane 7/3, affording 2.6 g of 1-(4-cyanobutyl)-4-(2-methoxy-phenyl) piperazine in 94.3% yield as a oil.
To a stirred solution of 10 ml of concentrated sulfuric acid was slowly added 2.75 g of 1-(4-cyanobutyl)-4-(2-methoxy-phenyl)piperazine and the resulting mixture was stirred first at 50°C for 30' then set aside at room temperature for 1 hour.
The reaction mixture was poured on ice, and after alkalization with concentrated ammonia solution, was extracted several times with dichloromethane.
After drying, the solvent was removed and the residue crystallized from diethyl ether affording 2.46 g of 1-(4-aminocarbonylbutyl)-4-(2-methoxy-phenyl)piperazine in 84% yield as white solid.
To a refluxing solution of 2.93 g of 1-(4-aminocarbonylbutyl)-4-(2-methoxy-phenyl)piperazine in 30
To a refluxing solution of 2.93 g of 1-(4-aminocarbonylbutyl)-4-(2-methoxy-phenyl)piperazine in 30 ml of anhydrous toluene was added portionwise 4.1 g of Lawesson's reagent.
After heating for 2 hours, the cold solution was filtered and washed 3 times with 1 M hydrochloric acid solution.
The acid extracts were basified with concentrate ammonia solution then extracted with ethyl acetate.
After drying, the crude mixture was chromatographed on silica gel eluting with chloroform/methanol 95/5 affording 1.85 g of 1-(4-aminothiocarbonylbutyl) -4-(2-methoxy-phenyl)piperazine in 65% yield.
A stirred solution of 3.2 g of 1-(4-aminothiocarbonylbutyl)-4-(2- methoxy-phenyl)piperazine and 3.82 g of 2-bromo-7-methoxy-1-tetralone- in 50 ml of diethyl ether was refluxed overnight.
After cooling, the solution was extracted several times with 1 M hydrochloric acid solution.
The acid phase was basified, extracted with ethyl acetate and dried.
The solvent was removed and the residue chromatographed on silica gel eluting with chloroform/methanol 98/2 affording 2.9 of the title compound in 65%yield.This by action of 1 M hydrochloric acid in methanol was converted in the corresponding hydrochloride, m.p.223-225°C.

### Example 2

### (2) 2-[(4-phenyl-piperazin-1-yl)-methyl]-4,5 dihydronaphtho[1,2-d]thiazole hydrocloride

Operating as in Example 1 but employing bromoacetonitrile instead of 5-bromopentane nitrile and 1-phenylpiperazine instead of 1-(2-methoxy-phenyl)piperazine and 2-bromo-1-tetralone instead of 2-bromo-6-methoxy-1-tetralone, the title compound was obtained in 78% yield, m. p.208-210°C.

### Example 3

### (3) 2-[2-(4-phenyl-piperazin-1-yl-ethyl]-4,5-dihydronaphtho[1,2-d]thiazole hydrocloride

Operating as in Example 2 but employing 2-bromopropane nitrile instead of bromoacetonitrile, the title compound was obtained in 56% yield, m.p.177-178°C.

### Example 4

### (4) 2-[3-(4-phenyl-piperazin-1-yl)-propyl]-4,5-dihydronaphtho[1,2-d]thiazole hydrocloride

Operating as in Example 2 but employing 3-bromobutyro-nitrile instead of bromoacetonitrile, the title compound was obtained in 47% yield, m.p.205-206°C.

### Example 5

### (5) 2-[4-(4-phenyl-piperazin-1-yl)-butyl]-4,5-dihydronaphtho[1,2-d]thiazole hydrocloride

Operating as in Example 2 but employing 4-bromovalero-nitrile instead of bromoacetonitrile, the title compound was obtained in 64% yield, m.p.208-210°C.

### Example 6

### (6) 7-Methoxy-2-{3-[4-(2-methoxy-phenyl) -piperazin-1-yl]-propyl}-4,5-dihydro-naphtho[1,2-d]thiazole hydrochloride

Operating as in Example 1 but employing 4-bromobutyro-nitrile instead of 5-bromovalero-nitrile and 1-phenylpiperazine instead of 1-(2-methoxy-phenyl)piperazine and 2-bromo-5-methoxy-1-tetralone instead of 2-bromo-6-methoxy-1-tetralone, the title compound was obtained in 34% yield, m. p.199-201°C.

### Example 7

### (7) 7-Methoxy-2-[4- (4-phenyl-piperazin-1-yl) -butyl]-4,5-dihydro-naphtho[1,2-d]thiazole hydrochloride

Operating as in Example 6 but employing 5-bromovalero-nitrile instead of 4-bromobutyro-nitrile, the title compound was obtained in 43% yield, m.p.200-202°C.

### Example 8

### (8) 7-Methoxy-2-{4-[4- (2-methoxy-phenyl) -piperazin-1-yl]-butyl}-4,5-dihydro-naphtho[1,2-d]thiazole hydrochloride

Operating as in Example 7 but employing 1-(2-methoxyphenyl)piperazine instead of 1-phenylpiperazine, the title compound was obtained in 37% yield, m.p.201-203°C.

### Example 9

### (9) 7-Methoxy-2-{4-[4-(2-pyridyl)-piperazin-1-yl]-butyl}-4,5-dihydro-naphtho[1,2-d]thiazole hydrochloride

Operating as in Example 7 but employing 1-(2-pyridyl)piperazine instead of 1-phenylpiperazine, the title compound was obtained in 48% yield, m.p.245-247°C.

### Example 10

### (10) 6-Methoxy-2-[4-(4-phenyl-piperazin-1-yl)-butyl]-4,5-dihydro-naphtho[1,2-d]thiazole hydrochloride

Operating as in Example 1 but employing 1-phenylpiperazine instead of 1-(2-methoxy-phenyl)piperazine, the title compound was obtained in 47% yield, m.p.221-222°C.

### Example 11

### (11) 5-Methoxy-2-[4-(4-phenyl-piperazin-1-yl)-butyl]-4,5-dihydro-naphtho[1,2-d]thiazole hydrochloride

Operating as in Example 10 but employing 2-bromo-7-methoxy-1-tetralone instead of 2-bromo-6-methoxy-1-tetralone, the title compound was obtained in 35% yield, m.p.195-197°C.

### Example 12

### (12) 5-Methoxy-2-{4-[4-(2-pyridyl)-piperazin-1-yl]-butyl}-4,5-dihydro-naphtho[1,2-d]thiazole hydrochloride

Operating as in Example 11 but employing 1-(2-pyridyl)piperazine instead of 1-phenylpiperazine, the title compound was obtained in 45% yield, m.p.219-221°C.

### Example 13

### (13) 5-Methoxy-2-{4-[4-(2-methoxy-phenyl)piperazin-1-yl]-butyl}-4,5-dihydro-naphtho[1,2-d]thiazole hydrochloride

Operating as in Example 11 but employing 1-(2-methoxyphenyl)piperazine instead of 1-phenylpiperazine, the title compound was obtained in 32% yield, m.p.207-209°C.

## Claims

1. An aryl or heteroaryl piperazine derivative of formula (I) 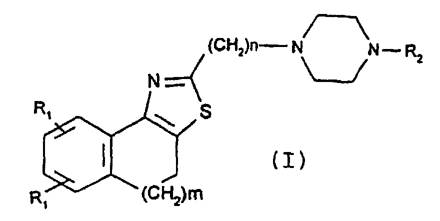 wherein
R₁ is hydrogen or a C₁-C₆ alkoxy group;
m is zero 1 or 2;
n is an integer from 1 to 4;
R₂ is an unsubstituted phenyl ring, a phenyl ring substituted by a C₁-C₃ alkoxy group, an unsubstituted pyridyl ring, an unsubstituted pyrimidinyl ring or an unsubstituted pyrazinyl ring.

2. A compound as claimed in claim 1 or 2, which is a hydrochloride salt.

3. A compound selected from the group consisting of:
(1) 6-Methoxy-2-{4-(4-(2-methoxy-phenyl)-piperazin-1-yl]-butyl}-4,5-dihydro-naphtho[1,2-d]thiazole hydrochloride;
(2) 2-[(4-phenyl-piperazin-1-yl)-methyl]-4,5 dihydro-naphtho[1,2-d]thiazole hydrocloride;
(3) 2-[2-(4-phenyl-piperazin-1-yl-ethyl]-4,5-dihydro-naphtho[1,2-d]thiazole hydrocloride;
(4) 2-[3-(4-phenyl-piperazin-1-yl)-propyl]-4,5-dihydro-naphtho[1,2-d]thiazole hydrocloride;
(5) 2-[4-(4-phenyl-piperazin-1-yl)-butyl]-4,5-dihydro-naphtho[1,2-d]thiazole hydrocloride;
(6) 7-Methoxy-2-{3-[4-(2-methoxy-phenyl)-piperazin-1-yl]-propyl}-4,5-dihydro-naphtho[1,2-d]thiazole hydrochloride;
(7) 7-Methoxy-2-[4-(4-phenyl-piperazin-1-yl)-butyl]-4,5-dihydronaphtho[1,2-d]thiazole hydrochloride;
(8) 7-Methoxy-2-{4-[4-(2-methoxy-phenyl)-piperazin-1-yl]-butyl}-4,5-dihydro-naphtho[1,2-d]thiazole hydrochloride;
(9) 7-Methoxy-2-{4-[4-(2-pyridyl)-piperazin-1-yl]-butyl}-4,5-dihydronaphtho[1,2-d]thiazole hydrochloride;
(10) 6-Methoxy-2-[4-(4-phenyl-piperazin-l-yl)-butyl]-4,5-dihydronaphtho[1,2-d]thiazole hydrochloride;
(11) 5-Methoxy-2-[4-(4-phenyl-piperazin-1-yl)-butyl]-4,5-dihydronaphtho[1,2-d]thiazole hydrochloride;
(12) 5-Methoxy-2-{4-[4-(2-pyridyl)-piperazin-1-yl]-butyl}-4,5-dihydronaphtho[1,2-d]thiazole hydrochloride; and
(13) 5-Methoxy-2-{4-[4-(2-methoxy-phenyl)-piperazin-1-yl]-butyl}-4,5-dihydro-naphtho[1,2-d]thiazole hydrochloride.

4. A process for preparing a compound as defined in claim 1, which process comprises:
a) condensing a compound of formula (II) with a compound of formula (III) 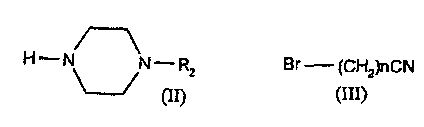 wherein n and R₂ are as defined in claim 1 so obtaining a compound of formula (IV) 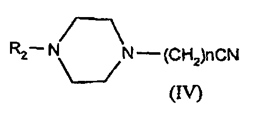 wherein n and R₂ are as defined above;
b) hydrolysing the compound of formula (IV) under acid conditions so as to obtain a compound of formula (V) 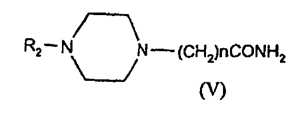 wherein n and R₂ are as defined above;
c) reacting the compound of formula (V) with P₂S₅ or the Lawesson reagent [2,4-bis(4-methoxyphenyl)-1,3-dithia-2,4-diphosphetane-2,4-disulfide], to obtain a compound of formula (VI) 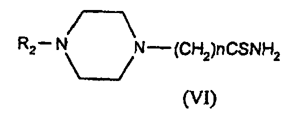 wherein R₂ and n are as defined above;
d) condensing the compound of formula (VI) with a compound of formula (VII) 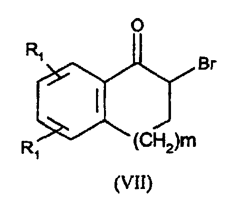
wherein R₁ is hydrogen, C₁-C₆ alkoxy, and m is zero or an integer from 1 to 2; and, if desired, converting the resulting compound of formula (I) into another compound of formula (I); and/or if desired converting a compound of formula (I) into a pharmaceutically acceptable salt thereof.

5. A pharmaceutical composition which comprises, as an active ingredient, a compound as defined in claim 1 in admixture with a pharmaceutically acceptable diluent and/or carrier.

6. A compound as defined in claim 1, for use in the treatment of a disorder depending on serotonin action at the level of 5-HT_{1A} receptor sites.

7. A compound as defined in claim 1, for use in the treatment of anxiety, depression, schizophrenia, pain, age associated memory impairment, Alzheimer's disease, a feeding or a sexual disorder.

8. A compound as defined in claim 1, for use in alleviating neuronal damage following cerebral ischemia.

## Patentansprüche

1. Aryl- oder Heteroarylpiperazinderivat der Formel (I): 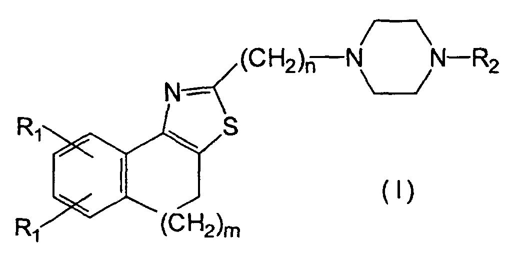 worin
R¹ Wasserstoff oder eine C₁₋₆-Alkoxygruppe ist;
m 0, 1 oder 2 ist;
n eine ganze Zahl von 1 bis 4 ist;
R₂ ein unsubstituierter Phenylring, ein mit einer C₁₋₃-Alkoxygruppe substituierter Phenylring, ein unsubstituierter Pyridylring, ein unsubstituierter Pyrimidinylring oder ein unsubstituierter Pyrazinylring ist.

2. Verbindung gemäss Anspruch 1, die ein Hydrochloridsalz ist.

3. Verbindung, die aus der Gruppe ausgewählt ist, die aus:
(1) 6-Methoxy-2-{4-[4-(2-methoxy-phenyl)-piperazin-1-yl]-butyl}-4,5-dihydro-naphtho[1,2-d]thiazolhydrochlorid;
(2) 2-[(4-Phenyl-piperazin-1-yl)-methyl]-4,5-dihydronaphtho[1,2-d]thiazol-hydrochlorid;
(3) 2-[2-(4-Phenyl-piperazin-1-yl)-ethyl]-4,5-dihydronaphtho[1,2-d]thiazol-hydrochlorid;
(4) 2-[3-(4-Phenyl-piperazin-1-yl)-propyl]-4,5-dihydronaphtho[1,2-d]thiazol-hydrochlorid;
(5) 2-[4-(4-Phenyl-piperazin-1-yl)-butyl]-4,5-dihydronaphtho[1,2-d]thiazol-hydrochlorid;
(6) 7-Methoxy-2-{3-[4-(2-methoxy-phenyl)-piperazin-1-yl]-propyl}-4,5-dihydro-naphtho[1,2-d]thiazolhydrochlorid;
(7) 7-Methoxy-2-[4-(4-phenyl-piperazin-1-yl)-butyl]-4,5-dihydro-naphtho[1,2-d]thiazol-hydrochlorid;
(8) 7-Methoxy-2-{4-[4-(2-methoxy-phenyl)-piperazin-1-yl]-butyl}-4,5-dihydro-naphtho[1,2-d]thiazol-Hydrochlorid;
(9) 7-Methoxy-2-{4-[4-(2-pyridyl)-piperazin-1-yl]-butyl}-4,5-dihydro-naphtho[1,2-d]thiazolhydrochlorid;
(10) 6-Methoxy-2-[4-(4-phenyl-piperazin-1-yl)-butyl]-4,5-dihydro-naphtho[1,2-d]thiazol-hydrochlorid;
(11) 5-Methoxy-2-[4-(4-phenyl-piperazin-1-yl)-butyl]-4,5-dihydro-naphtho[1,2-d]thiazol-hydrochlorid;
(12) 5-Methoxy-2-{4-[4-(2-pyridyl)-piperazin-1-yl]-butyl}-4,5-dihydro-naphtho[1,2-d]thiazolhydrochlorid; und
(13) 5-Methoxy-2-{4-[4-(2-methoxy-phenyl)-piperazin-1-yl]-butyl}-4,5-dihydro-naphtho[1,2-d]thiazolhydrochlorid
besteht.

4. Verfahren zur Herstellung einer Verbindung wie in Anspruch 1 definiert, wobei das Verfahren umfasst:
(a) Kondensieren einer Verbindung der Formel (II) mit einer Verbindung der Formel (III) : 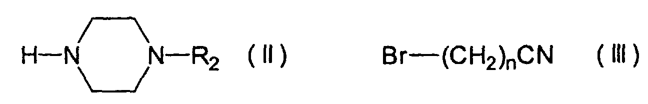 worin n und R₂ wie in Anspruch 1 definiert sind, um so eine Verbindung der Formel (IV) zu erhalten: 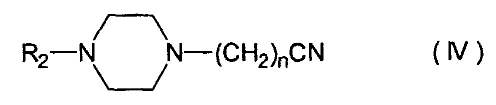 worin n und R2 wie oben definiert sind;
(b) Hydrolysieren der Verbindung der Formel (IV) unter sauren Bedingungen, um eine Verbindung der Formel (V) zu erhalten: 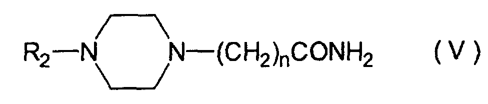 worin n und R₂ wie oben definiert sind;
(c) Umsetzen der Verbindung der Formel (V) mit P₂S₅ oder dem Lawesson-Reagens [2,4-Bis(4-methoxyphenyl)-1,3-dithia-2,4-diphosphetan-2,4-disulfid], um eine Verbindung der Formel (VI) zu erhalten: 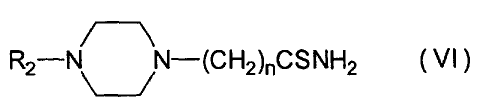 worin R₂ und n wie oben definiert sind;
(d) Kondensieren der Verbindung der Formel (VI) mit einer Verbindung der Formel (VII): 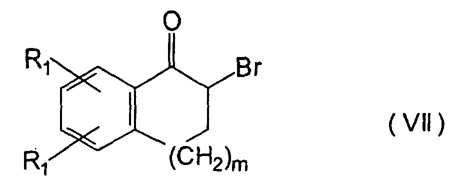
worin R₁ Wasserstoff oder C₁₋₆-Alkoxy ist und m 0 oder eine ganze Zahl von 1 bis 2 ist; und nach Wunsch Umwandeln der resultierenden Verbindung der Formel (I) zu einer anderen Verbindung der Formel (I); und/oder nach Wunsch Umwandeln einer Verbindung der Formel (I) zu einem pharmazeutisch akzeptablen Salz davon.

5. Pharmazeutische Zusammensetzung, die als aktiven Bestandteil eine Verbindung wie in Anspruch 1 definiert im Gemisch mit einem pharmazeutisch akzeptablen Verdünnungsstoff und/oder Träger umfasst.

6. Verbindung wie in Anspruch 1 definiert zur Verwendung in der Behandlung einer Störung, die von der Serotoninwirkung auf der Ebene der 5-HT_{1A}-Rezeptorstellen abhängt.

7. Verbindung wie in Anspruch 1 definiert zur Verwendung in der Behandlung von Angstzuständen, Depression, Schizophrenie, Schmerz, altersbedingter Gedächtnisbeeinträchtigung, Alzheimer-Krankheit, Nahrungszufuhr- oder Geschlechtsstörung.

8. Verbindung wie in Anspruch 1 definiert zur Verwendung in der Linderung von neuronaler Schädigung im Anschluss an zerebrale Ischämie.

## Revendications

1. Dérivé d'aryl ou hétéroaryl pipérazine de formule (I) 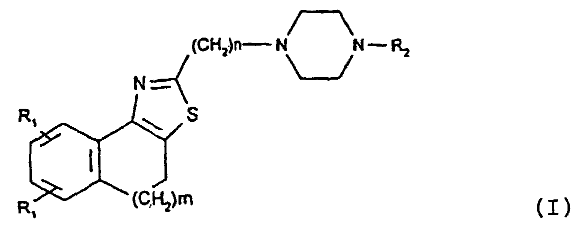 dans laquelle
R₁ est hydrogène ou un groupe alkoxy en C₁-C₆ ;
m est zéro, 1 ou 2 ;
n est un nombre entier de 1 à 4 ;
R₂ est un cycle phényle non substitué, un cycle phényle substitué par un groupe alkoxy en C₁-C₃, un cycle pyridyle non substitué, un cycle pyrimidinyle non substitué ou un cycle pyrazinyle non substitué.

2. Composé tel que revendiqué dans la revendication 1 ou 2, qui est un sel chlorhydrate.

3. Composé choisi dans le groupe constitué par :
(1) Chlorhydrate de 6-méthoxy-2-{4-[4-(2-méthoxyphényl)-pipérazin-1-yl]butyl}-4,5-dihydro-naphto[1,2-d]thiazole ;
(2) Chlorhydrate de 2-[(4-phényl-pipérazin-1-yl)méthyl]-4,5-dihydro-naphto[1,2-d]thiazole ;
(3) Chlorhydrate de 2-[2-(4-phényl-pipérazin-1-yl)-éthyl]-4,5-dihydro-naphto[1,2-d]thiazole ;
(4) Chlorhydrate de 2-[3-(4-phényl-pipérazin-l-yl)-propyl]-4,5-dihydro-naphto[1,2-d]thiazole ;
(5) Chlorhydrate de 2-[4-(4-phényl-pipérazin-l-yl)-butyl]-4,5-dihydro-naphto[1,2-d]thiazole ;
(6) Chlorhydrate de 7-méthoxy-2-{3-[4-(2-méthoxyphényl)-pipérazin-1-yl]-propyl}-4,5-dihydro-naphto[1,2-d]thiazole ;
(7) Chlorhydrate de 7-méthoxy-2-[4-(4-phényl-pipérazin-1-yl)-butyl]-4,5-dihydro-naphto[1,2-d]thiazole ;
(8) Chlorhydrate de 7-méthoxy-2-{4-[4-(2-méthoxyphényl)-pipérazin-1-yl]-butyl}-4,5-dihydro-naphto[1,2-d]thiazole ;
(9) Chlorhydrate de 7-méthoxy-2-{4-[4-(2-pyridyl)-pipérazin-1-yl]-butyl}-4,5-dihydro-naphto[1,2-d]thiazole ;
(10) Chlorhydrate de 6-méthoxy-2-[4-(4-phényl-pipérazin-1-yl)-butyl]-4,5-dihydro-naphto[1,2-d]thiazole ;
(11) Chlorhydrate de 5-méthoxy-2-[4-(4-phényl-pipérazin-1-yl)-butyl]-4,5-dihydro-naphto[1,2-d]thiazole ;
(12) Chlorhydrate de 5-méthoxy-2-{4-[4-(2-pyridyl)-pipérazin-1-yl]-butyl}-4,5-dihydro-naphto[1,2-d]thiazole ; et
(13) Chlorhydrate de 5-méthoxy-2-{4-[4-(2-méthoxyphényl)-pipérazin-1-yl]-butyl}-4,5-dihydro-naphto[1,2-d]thiazole.

4. Procédé pour préparer un composé tel que défini dans la revendication 1, lequel procédé comprend :
a) la condensation d'un composé de formule (II) avec un composé de formule (III) 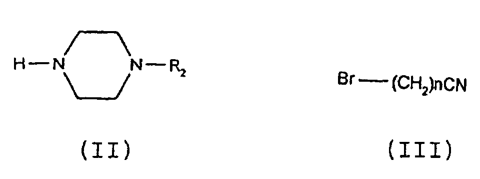 dans laquelle n et R₂ sont tels que définis dans la revendication 1, pour obtenir ainsi un composé de formule (IV) 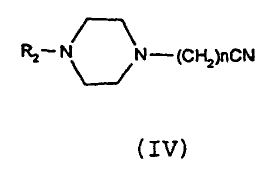 dans laquelle n et R₂ sont tels que définis ci-dessus ;
b) l'hydrolyse du composé de formule (IV) dans des conditions acides de façon à obtenir un composé de formule (V) 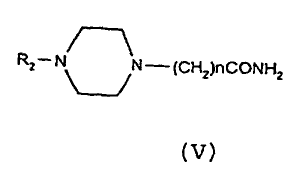 dans laquelle n et R₂ sont tels que définis ci-dessus ;
c) la réaction du composé de formule (V) avec P₂S₅ ou le réactif de Lawesson [2,4-bis(4-méthoxyphényl)-1,3-dithia-2,4-diphosphétane-2,4-disulfure], pour obtenir un composé de formule (VI) 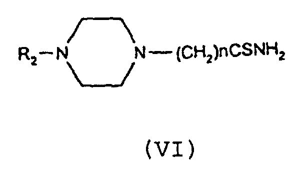 dans laquelle R₂ et n sont tels que définis ci-dessus ;
d) la condensation du composé de formule (VI) avec un composé de formule (VII) 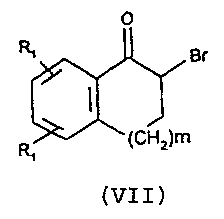 dans laquelle R₁ est hydrogène, alkoxy en C₁-C₆, et m est zéro ou un nombre entier de 1 à 2 ; et, si souhaité, la conversion du composé résultant de formule (I) en un autre composé de formule (I) ; et/ou, si souhaité, la conversion d'un composé de formule (I) en un sel pharmaceutiquement acceptable de celui-ci.

5. Composition pharmaceutique qui comprend, en tant qu'ingrédient actif, un composé tel que défini dans la revendication 1 en mélange avec un diluant et/ou un véhicule pharmaceutiquement acceptable.

6. Composé tel que défini dans la revendication 1, pour une utilisation dans le traitement d'une maladie dépendant d'une action de la sérotonine au niveau des sites des récepteurs 5-HT_{1A}.

7. Composé tel que défini dans la revendication 1, pour une utilisation dans le traitement de l'anxiété, de la dépression, de la schizophrénie, de la douleur, de l'affaiblissement de la mémoire associé à l'âge, de la maladie d'Alzheimer, d'une maladie liée à la nutrition ou d'une maladie sexuelle.

8. Composé tel que défini dans la revendication 1, pour une utilisation dans le soulagement du dommage neuronal qui suit une ischémie cérébrale.
